(19) Europäisches Patentamt
European Patent Office
Office européen des brevets

(11) Publication number: 0 167 050
A1

(12) # EUROPEAN PATENT APPLICATION

(21) Application number: 85107476.5

(22) Date of filing: 15.06.85

(51) Int. Cl.⁴: C 07 D 498/04
A 61 K 31/42
//(C07D498/04, 263:00, 205:00)

(30) Priority: 21.06.84 GB 8415924
20.11.84 GB 8429244

(43) Date of publication of application:
08.01.86 Bulletin 86/2

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: Harbridge, John Barry
53 Monson Road
Redhill Surrey, RH1 2EU(GB)

(72) Inventor: Brooks, Gerald
6 Worcester Road
Reigate Surrey, RH2 9HW(GB)

(74) Representative: Dayneswood, Trevor et al,
Beecham Pharmaceuticals Patent and Trade Mark
Department Biosciences Research Centre Great Burgh
Yew Tree Bottom Road
Epsom Surrey KT18 5XQ(GB)

(54) **Clavulanate compounds, their preparation and use and intermediates thereof.**

(57) Compounds of the general formula I:

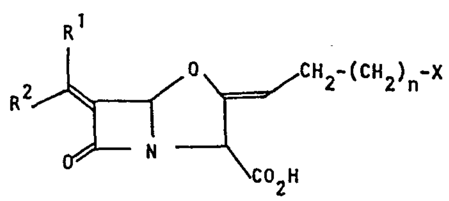

(I)

and their pharmaceutically acceptable salts and *in-vivo* hydrolysable esters,

in which

each of $R^1$ and $R^2$, which may be the same or different, denotes a hydrogen atom, an aryl group, or a heterocyclyl group;

X denotes a hydrogen atom, or a hydroxy, substituted hydroxy, mercapto, substituted mercapto, azido, cyano, halo, nitro, isothiocyanato, amino, substituted amino, aryl, or heterocyclyl group, or the residue of a carbon nucleophile; and

*n* denotes 0, 1 or 2.

are novel compounds with ß-lactamase inhibitory and antibacterial properties.

EP 0 167 050 A1

Croydon Printing Company Ltd.

NOVEL COMPOUNDS

TITLE MODIFIED see front page

This invention relates to β-lactam compounds, their preparation and their use, and in particular to a novel class of clavulanates, which have β-lactamase inhibitory and antibacterial properties. The compounds are therefore useful in the treatment of bacterial infections in humans and animals either alone or in combination with other antibiotics, especially in a synergistic composition in conjunction with other β-lactam antibacterial agents, such as penicillins and cephalosporins.

British Patent Specification No. GB 1,582,884 discloses compounds of the general formula (A):

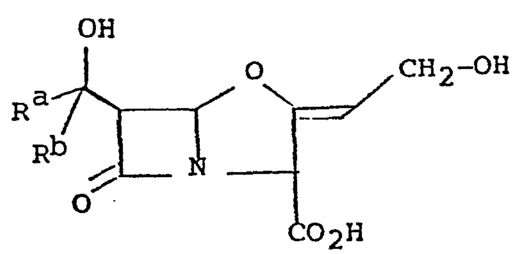

(A)

and salts and esters thereof,

in which

each of $R^a$ and $R^b$ denotes hydrogen or $(C_{1-4})$alkyl, or

$R^a$ and $R^b$ together denote part of a 5, 6 or 7-membered carbocyclic ring.

The present invention provides a compound of the general formula I:

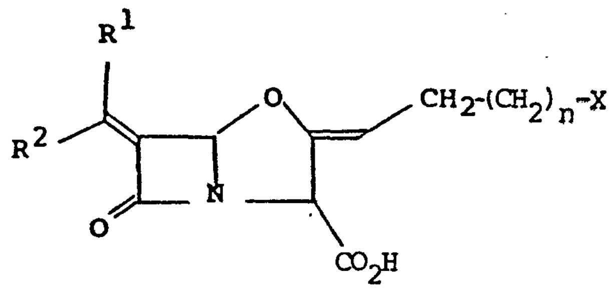

(I)

or a pharmaceutically acceptable salt or in-vivo hydrolysable ester thereof;

in which

each of $R^1$ and $R^2$ which may be the same or different, denotes a hydrogen atom, an aryl group or a heterocyclyl group;

X denotes a hydrogen atom, or a hydroxy, substituted hydroxy, mercapto, substituted mercapto, azido, cyano, halo, nitro, isothiocyanato, amino, substituted amino, aryl, or heterocyclyl (especially aromatic heterocyclyl) group, or the residue of a carbon nucleophile; and

$n$ denotes 0, 1 or 2.

The term 'heterocyclyl' as used herein includes aromatic and non-aromatic, single and fused rings containing up to four hetero-atoms in each ring selected from oxygen, nitrogen and sulphur, which rings may be unsubstituted or substituted by up to three

groups selected from halogen, $(C_{1-6})$alkyl, $(C_{1-6})$alkoxy, halo$(C_{1-6})$alkyl, hydroxy, amino, carboxy, $(C_{1-6})$alkoxycarbonyl, $(C_{1-6})$alkoxycarbonyl$(C_{1-6})$alkyl, aryl, $(C_{1-6})$alkylthio, arylthio, mercapto and oxo groups. The or each ring suitably has from 4 to 7, preferably 5 or 6, ring atoms. Suitably the heterocyclic ring or ring system is aromatic.

The term 'heteroaryl' as used herein means an aromatic heterocyclyl group as hereinbefore defined.

The term 'aryl' as used herein includes phenyl and naphthyl, which may be unsubstituted or substituted by up to five, preferably up to three, groups selected from halogen, $(C_{1-6})$alkyl, phenyl, $(C_{1-6})$alkoxy, halo$(C_{1-6})$alkyl, hydroxy, amino, nitro, carboxy, $(C_{1-6})$alkoxycarbonyl, $(C_{1-6})$alkoxycarbonyl$(C_{1-6})$alkyl, $(C_{1-6})$alkylcarbonyloxy, $(C_{1-6})$alkylcarbonyl, $(C_{1-6})$alkylthio, arylthio, and mercapto groups.

The term 'hydrocarbon' as used herein includes groups having up to 20 carbon atoms, advantageously up to 18 carbon atoms, suitably up to 10 carbon atoms, conveniently up to 6 carbon atoms. Suitable hydrocarbon groups include $(C_{1-6})$alkyl, $(C_{2-6})$alkenyl, $(C_{2-6})$alkynyl, $(C_{3-7})$cycloalkyl, $(C_{3-7})$cycloalkyl-$(C_{1-6})$alkyl, aryl, and aryl$(C_{1-6})$alkyl.

Suitably, one of $R^1$ and $R^2$ denotes a hydrogen atom and the other of $R^1$ and $R^2$ denotes an aryl group or an aromatic heterocyclyl group. Examples of suitable aryl groups denoted by $R^1$ and/or $R^2$ include unsubstituted and substituted phenyl groups. Examples of suitable heterocyclyl groups denoted by $R^1$ and/or $R^2$ include unsubstituted and substituted furyl, thienyl,

thiazolyl, isothiazolyl, oxazolyl, isoxazolyl, thiadiazolyl, oxadiazolyl, pyrazolyl, imidazolyl, triazolyl, tetrazolyl, pyridyl, pyrazinyl, pyrimidinyl, pyridazinyl, triazinyl, tetrazinyl, benzofuryl, indolyl, and benzotriazolyl groups.

In the compounds of the general formula (I), preferably n denotes 0.

Suitably X denotes a hydroxy or substituted hydroxy group. Examples of substituted hydroxy groups include the groups of the formulae

$-OR^5$, $-OCOR^5$, $-OCSR^5$, $-OCO_2R^5$, $-OCS_2R^5$, $-OCOSR^5$, $-OCSOR^5$, $-OSO_3H$, $-OPO_3H$, $-OSO_2R^5$ and $-OCONR^6R^7$

in which

$R^5$ denotes an unsubstituted or substituted hydrocarbon group having up to 20 carbon atoms or an unsubstituted or substituted heterocyclyl group, and

each of $R^6$ and $R^7$, which may be the same or different, denotes an unsubstituted or substituted hydrocarbon group having up to 20 carbon atoms.

Suitably $R^5$ denotes a $(C_{1-10})$alkyl, $(C_{2-10})$alkenyl, aryl$(C_{1-6})$alkyl, heterocyclyl$(C_{1-6})$alkyl, aryl$(C_{2-6})$alkenyl, $(C_{3-8})$cycloalkyl, aryl, heterocyclyl or $(C_{3-8})$cycloalkyl$(C_{1-6})$alkyl group, any of which groups mayng optionally be substituted. When $R^5$ denotes an aryl or heterocyclyl group, examples of suitable substituents include those listed above in the definitions of those terms. Otherwise suitable substituents for the group $R^5$, and also suitable

substituents for the groups $R^6$ and $R^7$, include amino, $(C_{1-6})$alkanoylamino, (mono or di)-$(C_{1-6})$alkylamino, hydroxy, $(C_{1-6})$alkoxy, mercapto, $(C_{1-6})$alkylthio, heterocyclylthio, arylthio, oxo, sulphamoyl, carbamoyl, amidino, guanidino, nitro, chloro, bromo, fluoro, cyano, carboxy, carboxy salts,carboxy esters, $(C_{1-6})$alkanoyloxy, arylcarbonyl and heterocyclylcarbonyl groups.

Preferably X denotes a $(C_{1-6})$alkoxy group, especially a methoxy group; a $(C_{1-6})$alkanoyloxy group, such as an acetoxy group; a $(C_{1-6})$alkylsulphonyloxy group, such as amethanesulphonyloxy group; or an arylsulphonyloxy group, such as a p-toluene-sulphonyloxy group.

Suitably X denotes a mercapto or substituted mercapto group. Examples of substituted mercapto groups include the groups of the formulae

$-SR^8$, $-SCOR^8$, $-SCSR^8$, $-SCO_2R^8$, $-SCS_2R^8$, $-SCOSR^8$, $-SCSOR^8$, $-SSO_2R^8$, $-SCONR^6R^7$, $-S(O)_2R^8$ and $-SSR^8$,

in which

$R^6$ and $R^7$ are defined as above, and

$R^8$ denotes an unsubstituted or substituted hydrocarbon group having up to 20 carbon atoms or an unsubstituted or substituted heterocyclyl group.

Suitably $R^8$ denotes a $(C_{1-10})$alkyl, $(C_{2-10})$alkenyl, aryl$(C_{1-6})$alkyl, heterocyclyl$(C_{1-6})$alkyl, aryl$(C_{2-6})$alkenyl, $(C_{3-8})$cycloalkyl$(C_{1-6})$alkyl, heterocyclyl, aryl, heteroaryl, heteroaryl$(C_{1-6})$alkyl or $(C_{3-8})$cycloalkyl group, any of which groups may

optionally be substituted. Suitable substituents for the group $R^8$ include those mentioned above with reference to the group $R^5$.

Suitably X denotes amino ($-NH_2$) or substituted amino group. Examples of substituted amino groups include

$-NHR^{12}$, $-NR^{12}R^{13}$, $-NR^{12}R^{13}R^{14}$, $-NHCZ^1R^{12}$, $-NHCZ^1Z^2R^{12}$, $-NHSO_2R^{12}$, $-NHCZ^1NR^{12}R^{13}$, $-N(CZ^1R^{14})R^{15}$, $-N(CZ^1R^{14})CZ^2R^{15}$, $-N(CZ^1R^{14})Z^2R^{15}$, $-N(CZ^1Z^2R^{14})R^{15}$ and $-N(CZ^1Z^2R^{14})CZ^1Z^2R^{15}$,

in which

each of $Z^1$ and $Z^2$, which may be the same or different, denotes an oxygen or sulphur atom, and

each of $R^{12}$, $R^{13}$, $R^{14}$ and $R^{15}$, which may be the same or different, denotes an unsubstituted or substituted hydrocarbon group having up to 20 carbon atoms, or an unsubstituted or substituted heterocyclyl group, or

$R^{12}$ and $R^{13}$ together with the nitrogen atom to which they are attached denote a heterocyclic ring containing from three to ten carbon atoms and from one to four heteroatoms selected from oxygen, nitrogen and sulphur, at least one of which heteroatoms is the afore-mentioned nitrogen atom.

Suitable groups denoted by $R^{12}$, $R^{13}$, $R^{14}$ and $R^{15}$ include those listed above as suitable groups $R^5$. Also, suitable substituents for the groups $R^{12}$, $R^{13}$, $R^{14}$ and $R^{15}$ include those mentioned above with reference to the group $R^5$.

Preferably X denotes an amino, $(C_{1-6})$alkylamino, benzylamino, $(C_{1-6})$alkanoylamino or di$(C_{1-6})$alkylamino group.

Suitably X denotes the residue of a carbon nucleophile and may, for example, be a group of the formula

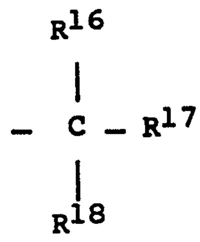

in which

$R^{16}$ denotes a group capable of producing a stabilised carbanion, and

$R^{17}$ denotes a hydrogen atom, an unsubstituted or substituted hydrocarbon group having up to 20 carbon atoms, an unsubstituted or substituted heterocyclyl group, or a group capable of producing a stabilised carbanion, and

$R^{18}$ denotes a hydrogen atom, an unsubstituted or substituted hydrocarbon group having up to 20 carbon atoms, or an unsubstituted or substituted heterocyclyl group.

A group capable of producing a stabilised carbanion may suitably be an electron withdrawing group. In particular, it may be a group of the formula

$-CZ^1R^{12}$ or $-CZ^1Z^2R^{12}$

as described above, in which $R^{12}$ is as defined above.

Suitable hydrocarbon and heterocyclyl groups $R^{17}$ and $R^{18}$ include those listed above as suitable groups $R^5$, and suitable substituents for such groups include those mentioned above with reference to the group $R^5$.

Suitable heterocyclyl groups denoted by X include tetrazolyl, triazolyl and trioxoimidazolidinyl groups, which may optionally be substituted by, for example, a $(C_{1-6})$alkoxycarbonyl group.

Suitably X denotes an azido or cyano group. Also, suitably X denotes a hydrogen atom or a halo moiety, for example iodo, bromo or chloro.

Pharmaceutically acceptable in-vivo hydrolysable esters (also referred to as 'metabolisable esters') of the compounds of the general formula I are those esters which hydrolyse in the human or animal body to produce the parent acid or its salt. Such esters may be identified by oral or intravenous administration to a test animal, and subsequent examination of the test animal's body fluids for the presence of the compound of the formula I or a salt thereof.

In some cases, the in-vivo hydrolysable ester moiety may constitute a link between two different active ingredient moieties, one of which is a compound according to the invention and the other of which may be another therapeutically active compound (for example another β-lactam antibiotic or β-lactamase inhibitor), such that on in-vivo hydrolysis of the ester moiety, the ester link breaks to give the two separate active compounds. The linked entity may be referred to as a 'mutual pro-drug'.

Suitable in-vivo hydrolysable ester groups include those of part-formulae (a), (b) and (c):

$A^1$
|
$-CO_2CH-O-CO-A^2$ (a)

$A^4$
|
$-CO_2-A^3-N$ (b)
|
$A^5$

$-CO_2CH_2-OA^6$ (c)

in which

$A^1$ denotes hydrogen, methyl, or phenyl;

$A^2$ denotes $(C_{1-6})$alkyl, $(C_{1-6})$alkoxy or phenyl; or

$A^1$ and $A^2$ together denote 1,2-phenylene, which may be unsubstituted or substituted by one or two methoxy groups;

$A^3$ denotes $(C_{1-6})$alkylene, which may be unsubstituted or substituted by a methyl or ethyl group;

each of $A^4$ and $A^5$ which may be identical or different, denotes $(C_{1-6})$alkyl; and

$A^6$ denotes $(C_{1-6})$alkyl.

Examples of suitable in vivo hydrolysable ester groups include acyloxyalkyl groups (e.g. acetoxymethyl, pivaloyloxymethyl, α-acetoxyethyl, and α-pivaloyloxyethyl), alkoxycarbonyloxyalkyl groups (e.g. ethoxycarbonyloxymethyl and α-ethoxycarbonyloxyethyl), acyloxyaralkyl (e.g. α-acetoxybenzyl), alkoxycarbonyloxyaralkyl (e.g. ethoxycarbonyloxybenzyl), dialkylaminoalkyl, especially di(lower alkyl)aminoalkyl (e.g. dimethylaminomethyl, diethylaminomethyl, dimethylaminoethyl, diethylaminoethyl), and lactone groups (e.g. phthalidyl and dimethoxyphthalidyl).

Suitable pharmaceutically acceptable salts of the 3-carboxylic acid group of the compound of formula I include metal salts, e.g. aluminium salts, alkali metal salts (e.g. sodium or potassium salts), alkaline earth metal salts (e.g. calcium or magnesium salts), ammonium salts, and substituted ammonium salts, for example those with lower alkylamines (e.g.triethylamine), hydroxy-lower alkylamines (e.g. 2-hydroxyethylamine), di(2-hydroxyethyl)amine or tri(2-hydroxyethyl)amine), cycloalkylamines (e.g. dicyclohexylamine), or with procaine, and also dibenzylamine, N,N-dibenzylethylenediamine, 1-ephenamine, N-ethylpiperidine, dibenzylpiperidine, N-benzyl-β-phenethylamine, dehydroabietylamine, N,N'-bishydroabietylethylene-diamine, glucamine, N-methylglucamine, bases of the pyridine type (e.g. pyridine, collidine and quinoline), and other amines which have been or could be used to form salts with penicillins.

Further suitable pharmaceutically acceptable salts are acid addition salts. Suitable acid addition salts of the compound or formula I include pharmaceutically

Suitable in-vivo hydrolysable ester groups include those of part-formulae (a), (b) and (c):

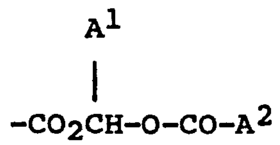

(a)

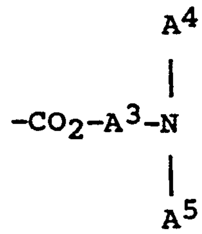

(b)

$-CO_2CH_2-OA^6$ (c)

in which

$A^1$ denotes hydrogen, methyl, or phenyl;

$A^2$ denotes $(C_{1-6})$alkyl, $(C_{1-6})$alkoxy or phenyl; or

$A^1$ and $A^2$ together denote 1,2-phenylene, which may be unsubstituted or substituted by one or two methoxy groups;

$A^3$ denotes $(C_{1-6})$alkylene, which may be unsubstituted or substituted by a methyl or ethyl group;

each of $A^4$ and $A^5$ which may be identical or different, denotes $(C_{1-6})$alkyl; and

$A^6$ denotes $(C_{1-6})$alkyl.

Examples of suitable in vivo hydrolysable ester groups include acyloxyalkyl groups (e.g. acetoxymethyl, pivaloyloxymethyl, α-acetoxyethyl, and α-pivaloyloxyethyl), alkoxycarbonyloxyalkyl groups (e.g. ethoxycarbonyloxymethyl and α-ethoxycarbonyloxyethyl), acyloxyaralkyl (e.g. α-acetoxybenzyl), alkoxycarbonyloxyaralkyl (e.g. ethoxycarbonyloxybenzyl), dialkylaminoalkyl, especially di(lower alkyl)aminoalkyl (e.g. dimethylaminomethyl, diethylaminomethyl, dimethylaminoethyl, diethylaminoethyl), and lactone groups (e.g. phthalidyl and dimethoxyphthalidyl).

Suitable pharmaceutically acceptable salts of the 3-carboxylic acid group of the compound of formula I include metal salts, e.g. aluminium salts, alkali metal salts (e.g. sodium or potassium salts), alkaline earth metal salts (e.g. calcium or magnesium salts), ammonium salts, and substituted ammonium salts, for example those with lower alkylamines (e.g.triethylamine), hydroxy-lower alkylamines (e.g. 2-hydroxyethylamine), di(2-hydroxyethyl)amine or tri(2-hydroxyethyl)amine), cycloalkylamines (e.g. dicyclohexylamine), or with procaine, and also dibenzylamine, N,N-dibenzylethylenediamine, 1-ephenamine, N-ethylpiperidine, dibenzylpiperidine, N-benzyl-β-phenethylamine, dehydroabietylamine, N,N'-bishydroabietylethylene-diamine, glucamine, N-methylglucamine, bases of the pyridine type (e.g. pyridine, collidine and quinoline), and other amines which have been or could be used to form salts with penicillins.

Further suitable pharmaceutically acceptable salts are acid addition salts. Suitable acid addition salts of the compound or formula I include pharmaceutically

acceptable inorganic acid addition salts, for example sulphate, nitrate, phosphate, borate, hydrochloride and hydrobromide, and also pharmaceutically acceptable organic acid addition salts, for example acetate, tartrate, maleate, citrate, succinate, benzoate, ascorbate, α-keto glutarate, α-glycerophosphate, and glucose-1-phosphate. Preferably the acid addition salt is a hemisuccinate, hydrochloride, α-ketoglutarate, α-glycerophosphate or glucose-1-phosphate, especially the hydrochloride salt.

The compounds of the general formula I and their salts may exist in hydrated or non-hydrated form.

The compounds of the general formula I and also the salts and esters thereof may exist in two optically active forms and it is to be understood that both such forms as well as racemic mixtures thereof are embraced by the present invention. The preferred form is that of the formula IA:

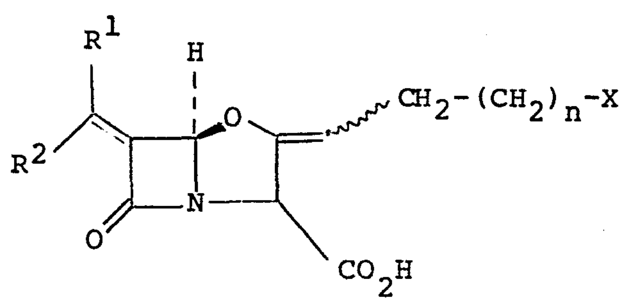

IA

in which $R^1$, $R^2$, X and n are defined as above.

The compounds of the general formula I may also have E or Z stereochemistry about the double bond at the 2-position, as shown by formula IB and IC respectively:

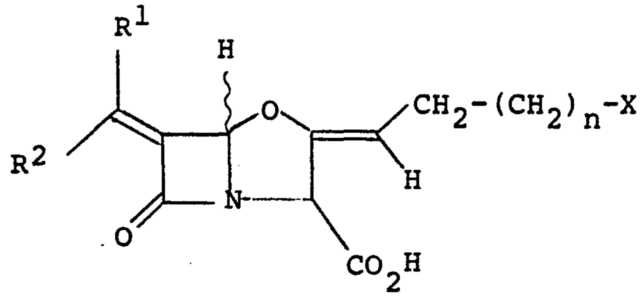

IB

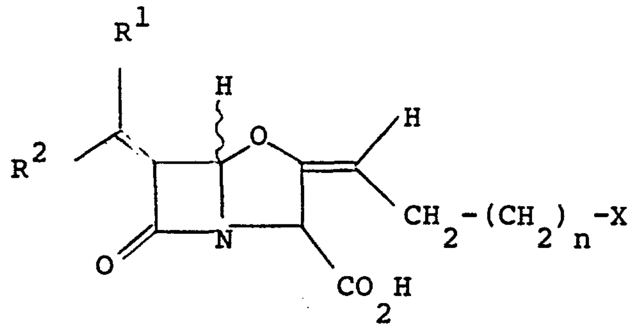

IC

Furthermore, in the event that $R^1$ and $R^2$ are different from one another, the compounds of the general formulae I, IA, IB and IC have E or Z stereochemistry about the double bond at the 6-position. It is generally thought to be advantageous that $R^1$ denotes an aryl or aromatic heterocyclyl group and $R^2$ denotes a hydrogen atom.

Examples of individual compounds according to the invention include:

6-E-benzylidene-9-O-methylclavulanate,

6-Z-benzylidene-9-O-methylclavulanate,

6-E-2'-furylmethylene-9-O-methylclavulanate,

and
6-Z-2'-furylmethylene-9-O-methylclavulanate,

as well as pharmaceutically acceptable salts and *in-vivo* hydrolysable esters thereof.

A compound of the general formula I, or a salt or ester thereof, may be prepared by eliminating the elements of a compound of the general formula II:

H-Y II

from a compound of the general formula

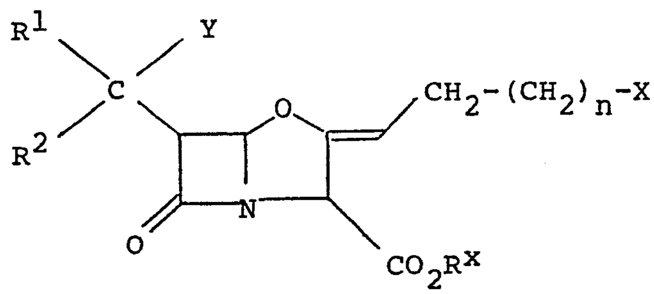

III

in which

$R^1$, $R^2$, X and *n* are defined as above,

Y denotes a hydroxy group or a leaving group, and

$R^x$ denotes hydrogen or a carboxyl-blocking group,

any reactive groups in X optionally being protected,

and thereafter if necessary or desired:

i) removing any protecting group on the group X; and/or

ii) converting one group X to a different group X; and/or

iii) removing any carboxyl-blocking group $R^x$; and/or

iv) converting the product into the free acid or into a pharmaceutically acceptable salt or in-vivo hydrolysable ester.

The conversion of one group X to another such group can be carried out by any suitable known methods appropriate to the X groups involved.

The compound of the general formula III are novel and as such form part of the invention. They may have α- or β-stereochemistry at the C-6 carbon atom or may be a mixture of both isomers.

A carboxyl-blocking group $R^x$ (also referred to as a carboxyl-protecting group) is suitably a group that can readily be removed at a later stage of the preparation process.

Examples of suitable carboxyl-blocking derivatives that may form the group $-CO_2R^x$ include salt, ester, and anhydride derivatives of the carboxylic acid.

The salts may be organic or inorganic and need not be pharmaceutically acceptable. Examples of suitable salt-forming groups $R^x$ include inorganic salts, for example alkali metal atoms (e.g. lithium, sodium and potassium), other metal atoms, tertiary amino groups (e.g. tri-lower-alkylamino, N-ethylpiperidino, 2,6-lutidino, pyridino, N-methylpyrrolidino, and dimethylpiperazino groups). A preferred salt-forming group $R^x$ is the triethylamino group.

An ester-forming group $R^x$ is advantageously one that can be removed under conventional conditions. Examples of suitable ester-forming groups $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, allyl, acetonyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluene-sulphonylethyl, and methoxymethyl groups, and also silyl, stannyl and phosphorus-containing groups, and oxime radicals of formula $-N=CHR^o$ in which $R^o$ denotes aryl or heterocyclyl. Furthermore, the ester-forming group $R^x$ may be an in-vivo hydrolysable ester group including, in particular, those listed above.

The free carboxyl group may be regenerated from any of the above esters by usual methods appropriate to the particular $R^x$ group, for example, by acid-catalysed, base-catalysed or enzymically-catalysed hydrolysis, or by hydrogenation. The hydrolysis must of course be carried out under conditions in which the groups on the rest of the molecule are stable.

When it is desired to produce a compound of the general formula I in the form of a free acid or in the form of a salt, by a process according to the invention, it is generally advantageous to use a compound in which $R^x$ denotes a carboxyl-blocking group. When it is desired to produce a compound of the general formula I in the form of a pharmaceutically acceptable ester, it is generally convenient to use a compound in which $R^x$ denotes the desired ester group.

The process step according to the invention involves the elimination of the elements of a compound H-Y from a compound of the general formula III, in which Y denotes a hydroxy group or a leaving group.

In the case where Y denotes a hydroxy group, the compound of the formula H-Y being eliminated is water and the elimination reaction is a dehydration reaction, which may suitably be carried out by treating a compound of the general formula III with a compound of the general formula IV:

$$R^{21}O_2C-N=N-CO_2R^{22} \qquad \text{IV}$$

in which each of $R^{21}$ and $R^{22}$, which may be identical or different, denotes aryl, $(C_{1-6})$alkyl or aryl$(C_{1-6})$alkyl,

and a with compound of the general formula V:

$$R^{23}(O)_a - \underset{\substack{| \\ (O)_cR^{25}}}{P} - (O)_bR^{24} \qquad \text{V}$$

in which

each of <u>a</u>, <u>b</u> and <u>c</u>, which may be identical or different, denotes 0 or 1, and

each of $R^{23}$, $R^{24}$ and $R^{25}$, which may be identical or different, denotes aryl, $(C_{1-6})$alkyl or aryl$(C_{1-6})$alkyl.

In the compounds of the general formula IV, $R^{21}$ and $R^{22}$ are preferably selected from methyl, ethyl, propyl, butyl, phenyl, and benzyl, the ethyl and t-butyl, groups being preferred. Advantageously, $R^{21}$ and $R^{22}$ may be identical. A preferred compound of the general formula IV is diethyl azodicarboxylate.

Preferred compounds of the general formula V include triarylphosphines and trialkylphosphites. Preferred groups $R^{23}$, $R^{24}$ and $R^{25}$ include methyl, ethyl, n-propyl, n-butyl, benzyl, phenyl and methoxyphenyl. Advantageously, $R^{23}$, $R^{24}$ and $R^{25}$ are all identical. A preferred compound of the general formula V is triphenylphosphine.

Advantageously, approximately two equivalents of each the compounds of the general formulae IV and V are used per mole of the compound of the general formula III.

The dehydration reaction may suitably be carried out at a non-extreme temperature, for example a temperature of from -20°C to +100°C. It may be convenient to begin the reaction at a depressed temperature, for example 0°C, and then to allow the temperature to rise to about room temperature.

The reaction may suitably be carried out in an inert aprotic organic solvent. Suitable solvents include tetrahydrofuran, dioxane, ethyl acetate, benzene, and dichloromethane.

In the cases where Y, in general formula III, denotes a leaving group, which will hereinafter be referred to as $Y^1$, it may suitably be a halogen atom or a group of one of the formulae

$-O-SO_2-(O)_n-R^{26}$ VIA

$-O-CO-(O)_n-R^{26}$ VIB or

$-O-PO-(OR^{27})_2$ VIC

in which

$\underline{n}$ denotes 0 or 1,

$R^{26}$ denotes $(C_{1-6})$alkyl, aryl or aryl$(C_{1-6})$alkyl,

and

$R^{27}$ denotes $(C_{1-6})$alkyl or aryl.

Preferred groups of formula VIA and VIB are those in which $\underline{n}$ denotes zero and $R^{26}$ denotes $(C_{1-6})$alkyl, especially the methanesulphonyl and acetoxy groups.

The elimination of the elements of a compound of the general formula II in which Y denotes a leaving group $Y^1$ from a compound of the general formula III, may suitably be effected by treating the compound of the general formula III with a base in an aprotic medium.

Suitable bases for that purpose include, for example, powdered inorganic bases, for example alkali metal carbonates, bicarbonates, hydroxides, and hydrides (e.g. powdered potassium carbonate), and also organic bases of low nucleophilicity, for example 1,8-diazabicyclo[5.4.0]undec-7-ene, and pyridine.

Suitable solvents for use as the aprotic medium in this reaction include,for example, dimethylformamide, hexamethylphosphoramide, dichloromethane, and tetrahydrofuran.

The elimination may suitably be effected at a low temperature, for example a temperature of from -20°C to +70°C, advantageously from +10°C to +25°C, preferably at ambient temperature.

The compounds of the general formula III in which Y denotes a leaving group $Y^1$ may suitably be prepared from the corresponding compound in which Y denotes a hydroxy group by replacing the hydroxy group by a leaving group $Y^1$ in a manner known *per se*.

A compound of the general formula III in which Y denotes a hydroxy group may suitably be obtained by reacting a compound of the general formula VII:

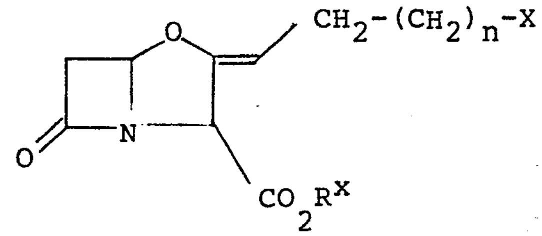

VII

in which X and $R^x$ are defined as above with a compound of the general formula VIII:

$$R^1 - CO - R^2 \qquad \text{VIII}$$

in which $R^1$ and $R^2$ are defined as above, in the presence of a base, for example lithium hexamethyldisilazide.

A compound of the general formula VII may be derived from clavulanic acid described in our British Patent No. GB 1,508,977, by conventional methods.

The compounds according to the invention have β-lactamase inhibitory and antibacterial properties, and are useful for the treatment of infections in animals, especially mammals, including humans, in particular in humans and domesticated (including farm)animals. The compounds may be used, for example, for the treatment of infections of, *inter alia*, the respiratory tract, the urinary tract, and soft tissues, especially in humans.

The compounds may be used for the treatment of infections caused by strains of, for example, *Staphylococcus aureus*, *Klebsiella aerogenes*, *Escherichia coli*, *Proteus sp*., and *Bacteroides fragilis*. The organism believed to be most readily treated by an antibacterially effective amount of a compound according to the invention is *Staphylococcus aureus*. The other organisms named are more readily treated by using a compound according to the invention in admixture or conjunction with a penicillin, cephalosporin or other β-lactam antibiotic, which can often result in a synergistic effect, because of the β-lactamase inhibitory properties of the compounds according to the invention. In such cases, the compound according to the invention and the other β-lactam antibiotic can be administered separately or in the form of a single composition containing both active ingredients as discussed in more detail below.

The compounds according to the invention are suitably provided in substantially pure form, for example at least 50% pure, advantageously at least 75% pure, preferably at least 95% pure, especially at least 98% pure, all percentages being calculated as weight/weight. An impure or less pure form of a compound according to the invention may, for example,

be used in the preparation of a more pure form of the same compound or of a related compound (for example, a corresponding salt, ester or free acid) suitable for pharmaceutical use. Although the purity of any compound used as an intermediate may be less critical than that of a compound used as a final product, for example one used directly for pharmaceutical use (for example in a composition according to the invention as described below), nevertheless such an intermediate compound is advantageously provided in substantially pure form. It is generally advantageous to provide the compounds according to the invention in crystalline form. The free acids and salts according to the invention may be in hydrated or non-hydrated form.

The present invention provides a pharmaceutical composition comprising a compound according to the invention that is to say, a compound of the general formula I or a pharmaceutically acceptable salt or *in-vivo* hydrolysable ester thereof, and a pharmaceutically acceptable carrier.

The present invention also provides a method of treating bacterial infections in animals, especially in humans and in domesticated mammals (including farm mammals), which comprises administering a compound or composition according to the invention to the animal. Such administration may advantageously be effected in conjunction with the prior, simultaneous or subsequent administration of a penicillin, cephalosporin or other β-lactam antibiotic.

The compositions of the invention may be in a form adapted for oral, topical or parenteral use and may be used for the treatment of infection in animals

especially mammals, including humans, in particular in humans and domesticated animals (including farm animals).

The compositions of the invention may, for example, be made up in the form of tablets, capsules, creams, syrups, suspensions, solutions, reconstitutable powders, and sterile forms suitable for injection or infusion. Such compositions may contain conventional pharmaceutically acceptable materials, for examplediluents, binders, colours, flavours, preservatives, and disintegrants, in accordance with conventional pharmaceutical practice in manner well understood by those skilled in the art of formulating antibiotics.

It can be particularly advantageous for the compounds according to the invention to be administered to a patient by injection or infusion. That method of administration has the advantage of rapidly resulting in high blood levels of the active ingredient compound being administered. Accordingly, in one preferred form of the composition according to the invention, a compound according to the invention is present in sterile form, including in sterile crystalline form. A further preferred form of the composition according to the invention, is one in which the composition is in injectable or infusable form.

One injectable or infusable form of the composition according to the invention is an injectable or infusable solution, which suitably comprises a solution of a compound according to the invention in a sterile pyrogen-free liquid, for example water or aqueous ethanol.

A further injectable or infusable form of the composition according to the invention is an injectable or infusable suspension, in which case the compound according to the invention is advantageously present in finely particulate form. The suspension may be an aqueous suspension in, for example, sterile water or sterile saline, which may additionally include a suspending agent, for example polyvinylpyrrolidone. Alternatively, the suspension may be an oily suspension in a pharmaceutically acceptable oil suspending agent, for example arachis oil.

A composition according to the invention may be in unit dosage form, for example unit dosage form for oral administration, topical administration, or parenteral administration (including administration by injection or infusion).

A composition according to the invention may comprise a compound according to the invention as the sole active ingredient or therapeutic agent, or it may also comprise one or more additional active ingredients or therapeutic agents, for example a penicillin, cephalosporin or other β-lactam antibiotic, or pro-drug thereof. A composition comprising a compound according to the invention and another active ingredient or therapeutic agent, especially a penicillin, cephalosporin or other β-lactam antibiotic, or pro-drug thereof, can show enhanced effectiveness, and in particular can show a synergistic effect.

Penicillins, cephalosporins and other β-lactam antibiotics suitable for co-administration with the compounds according to the invention - whether by separate administration or by inclusion in the

compositions according to the invention - include both those known to show instability to or to be otherwise susceptible to β-lactamases and also those known to have a degree of resistance to β-lactamases.

Examples of penicillins suitable for co-administration with the compounds according to the invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other known penicillins. The penicillins may be used in the form of pro-drugs thereof, for example as in-vivo hydrolysable esters, for example the acetoxymethyl, pivaloyloxymethyl, α-ethoxycarbonyloxyethyl and phthalidyl esters of ampicillin, benzylpenicillin and amoxycillin; as aldehyde or ketone adducts of penicillins containing a 6α-aminoacetamido side chain (for example hetacillin, metampicillin and analogous derivatives of amoxycillin); and as α-esters of carbenicillin andticarcillin, for example the phenyl and indanyl α-esters.

Examples of cephalosporins that may be co-administered with the compounds according to the invention include, cefatrizine, cephaloridine, cephalothin, cefazolin, cephalexin, cephacetrile, cephapirin, cephamandole nafate, cephradine, 4-hydroxycephalexin, cephaloglycin, cefoperazone, cefsulodin, ceftazidime, cefuroxime, cefmetazole, cefotaxime, ceftriaxone, and other known cephalosporins, all of which may be used in the form of pro-drugs thereof.

0167050

Examples of β-lactam antibiotics other than penicillins and cephalosporins that may be co-administered with the compounds according to the invention include aztreonam, latamoxef (Moxalactam - Trade Mark), and other known β-lactam antibiotics, all of which may be used in the form of pro-drugs thereof.

In the compositions according to the invention, the compound according to the invention and the penicillin, cephalosporin or other β-lactam antibiotic may be linked by means of an *in-vivo* hydrolysable ester group, in the form of a mutual pro-drug.

Some penicillins and cephalosporins that may be included in the compositions according to the invention may not be suitable for oral administration, in which case the composition will be in a form suitable for parenteral or topical administration.

Particularly suitable penicillins for co-administration with the compounds according to the invention include ampicillin, amoxycillin, carbenicillin, piperacillin, azlocillin, mezlocillin, and ticarcillin. Such penicillins may be used in the form of their pharmaceutically acceptable salts, for example their sodium salts. Alternatively, ampicillin or amoxycillin may be used in the form of fine particles of the zwitterionic form (generally as ampicillin trihydrate or amoxycillin trihydrate) for use in an injectable or infusable suspension, for example, in the manner hereinbefore described in relation to the compounds according to the invention. Amoxycillin, for example in the form of its sodium salt or the trihydrate, is particularly preferred for use in synergistic compositions according to the invention.

Particularly suitable cephalosporins for co-administration with the compounds according to the invention include cephaloridine, cefoperazone and cefazolin, which may be used in the form of their pharmaceutically acceptable salts, for example their sodium salts.

A compound according to the invention may be administered to the patient in an antibacterially effective amount or, when a compound according to the invention is being used in conjunction with a penicillin, cephalosporin, or other β-lactam antibiotic, it may be used in a synergistically effective amount.

The compounds according to the invention may suitably be administered to the patient at a daily dosage of from 0.7 to 15 mg/kg of body weight. For an adult human (of approximately 70 kg body weight), from 50 to 3000 mg, preferably from 100 to 1000 mg, of a compound according to the invention may be adminstered daily, suitably in from 1 to 6, preferably from 2 to 4, separate doses. Higher or lower dosages may, however, be used in accordance with clinical practice. In particular, higher doses may be used for the treatment of more severe systemic infections or infections of particularly intransigent organisms.

When the compositions according to the invention are presented in unit dosage form, each unit dose may suitably comprise from 25 to 1000 mg, preferably from 50 to 500 mg, of a compound according to the invention. Each unit dose may, for example, be 62.5, 100, 125, 150, 200 or 250 mg of a compound according to the invention.

When the compounds according to the invention are co-administered with a penicillin, cephalosporin or other β-lactam antibiotic, the ratio of the amount of the compound according to the invention to the amount of the other β-lactam antibiotic may vary within a wide range. The said ratio may, for example, be from 10:1 to 1:10; more particularly, it may, for example, be from 3:1 to 1:6 (weight:weight, based on pure free antibiotic equivalent).

The amount of penicillin or cephalosporin or other β-lactam antibiotic in a synergistic composition according to the invention will normally be approximately similar to the amount in which it is conventionally used *per se*, for example from about 50 mg, advantageously from about 62.5 mg, to about 3000 mg per unit dose, more usually about 125, 250, 500 or 1000 mg per unit dose.

An example of a particularly suitable composition according to the invention is one comprising from 150 to 1000 mg, preferably from 200 to 700 mg, of amoxycillin or ampicillin or a pro-drug thereof, in admixture or conjunction with from 25 to 500 mg, preferably from 50 to 250 mg, of a compound according to the invention, per unit dose. In such a composition, the amoxycillin may suitably be in the form of its trihydrate or sodium salt; the ampicillin may suitably be in the form of ampicillin trihydrate, ampicillin anhydrate, sodium ampicillin, hetacillin, pivampicillin hydrochloride, bacampicillin hydrochloride or talampicillin hydrochloride; and the compound according to the invention may most suitably be in crystalline form. Such composition may be in a form suitable for oral or parenteral use, except when it comprises an *in-vivo* hydrolysable ester of

ampicillin or amoxycillin in which case the composition should not normally be intended for parenteral administration.

A further example of a particularly suitable composition according to the invention is one comprising from 200 to 2000 mg of carbenicillin or ticarcillin or a pro-drug thereof, in admixture or conjunction with from 50 to 500 mg, preferably from 75 to 250 mg, of a compound according to the invention, per unit dose. In such a composition, the carbenicillin or ticarcillin may most suitably be in the form of its di-sodium salt, and the compound according to the invention may most suitably be in crystalline form. When the composition contains the carbenicillin or ticarcillin in the form of a di-salt, it is most suitably presented in a form suitable for parenteral administration.

No toxicological effects are indicated when the compounds according to the invention are administered within the above-mentioned dosage ranges.

The indications for treatment include respiratory tract and urinary tract infections in humans.

The following examples illustrate the present invention.

Example 1

Benzyl 6-R-6-(α-hydroxybenzyl)-9-O-methyl clavulanate

A mixture of tetrahydrofuran (200 ml, distilled from lithium aluminium hydride), hexamethylphosphoramide (20 ml) and hexamethyl disilazane (2.8 ml) was stirred under nitrogen at -70°C. To this was added a solution of n-butyl lithium in hexane (7.12 ml of a 1.86M solution) and after 15 minutes the mixture was treated dropwise over 2 minutes with a solution of benzyl 9-O-methyl clavulanate (2.0 g) in distilled tetrahydrofuran (10 ml). After a further 15 minutes the solution was treated dropwise over 2 minutes with a solution of benzaldehyde (.71 ml) in distilled tetrahydrofuran (5 ml). The resulting mixture was stirred at -70°C for 45 minutes, treated with a mixture of acetic acid (2.5 ml) and water (5 ml), then diluted with ethyl acetate (300 ml). After warming to room temperature, saturated brine (200 ml). was added, the mixture was shaken and the layers separated. The organic layer was dried over $MgSO_4$, filtered, and the filtrate was evaporated under reduced pressure. The residue was chromatographed on silica, eluting with ethyl acetate/hexane 1:2. Appropriate fractions were combined and evaporated under reduced pressure. The residue crystallised from ethyl acetate/ hexane to provide benzyl 6-R-6(α-hydroxybenzyl)-9-O-methyl clavulanate (500 mg) as colourless rectangular plates: m.p. 134 - 6°C; $\nu_{max.}$ (Nujol) 3290, 1805, 1795(s), 1735, 1690 $cm^{-1}$; δ ($CDCl_3$) 2.68 (1H, d, J 4 Hz), 3.15 (3H, s), 3.40 (1H, d, J 6 Hz), 3.7 - 4.1 (2H, m), 4.71 (1H, dt, J 7.5 and 1.5 Hz), 5.0 - 5.2 (4H, m), 5.75 (1H, s), 7.27 and 7.31 (10H, 2s). (Found: C 67.9, H 5.5, N 3.4%. $C_{23}H_{23}NO_6$ requires C 67.6, H 5.6, N 3.4%).

Example 2

Methoxymethyl 6-R-6-(α-hydroxybenzyl)-9-O-methyl clavulanate and methoxymethyl 6-S-6-(α-hydroxybenzyl)-9-O-methyl clavulanate

A mixture of tetrahydrofuran (200 ml, distilled from $LiAlH_4$), hexamethylphosphoramide (20 ml) and hexamethyldisilazane (3.29 ml) was stirred at -70°C under nitrogen and treated with a solution of n-butyl lithium in hexane (8.4 ml of a 1.86M solution). After 15 minutes the mixture was treated dropwise over 2 minutes with a solution of methoxymethyl 9-O-methyl clavulanate (2 g) in distilled tetrahydrofuran (10 ml). After a further 15 minutes a solution of benzaldehyde (.864 ml) in tetrahydrofuran (5 ml) was added. The mixture was stirred at -70°C for 45 minutes, treated with a mixture of acetic acid (2.5 ml) and water (5 ml) and warmed to room temperature. Ethyl acetate (300 ml) and saturated brine (200 ml) were added, the mixture was shaken and the layers separated. The organic layer was dried over $MgSO_4$, filtered, and the filtrate evaporated under reduced pressure. The residue was chromatographed on silica, eluting with ethyl acetate/hexane 1:2. Fractions containing the less polar component were combined and evaporated to provide methoxymethyl 6-R-6-(α-hydroxybenzyl)-9-O-methyl clavulanate as a gum (.91g): $\nu_{max.}$ ($CHCl_3$) 3600, 3380, 1790, 1750, 1690 $cm^{-1}$; δ ($CDCl_3$) 2.78 (1H, broad s), 3.20 (3H, s), 3.40 (3H, s), 3.52 (1H, d, J 5.5 Hz), 3.93 (2H, d, J 7.5 Hz), 4.78 (1H, dt, J 7.5 and 1 Hz), 5.0 - 5.4 (4H, m), 5.76 (1H, s), 7.33 (5H, s).

Fractions containing the more polar component were combined and evaporated to provide methoxymethyl 6-S-6-(α-hydroxy benzyl)-9-O-methyl clavulanate as a gum (.17 g): $\nu_{max}$ ($CHCl_3$) 3580, 3380, 1790, 1750, 1690 $cm^{-1}$; δ ($CDCl_3$) 3.04 (1H, d, J 3 Hz), 3.36 (3H, s), 3.48 (3H,s), 3.87 (1H, dd, J 8 and 3 Hz), 3.99 (1H, dd, H 12 and 7 Hz), 4.18 (1H, dd, J 12 and 7 Hz), 5.01 (1H, t, J 7 Hz), 5.1 - 5.2 (2H, m), 5.31 (2H, ABq), 5.63 (1H, d, J 3 Hz), 7.3 - 7.6 (5H, m).

Example 3

Sodium 6-R-6(α-hydroxybenzyl)-9-0-methyl clavulanate

To a solution of benzyl 6-R-6-(α-hydroxybenzyl)-9-O-methyl clavulanate (100 mg) in distilled tetrahydrofuran (8 ml) was added 10% Pd/C (35 mg). The mixture was shaken under hydrogen at atmospheric pressure for 15 minutes and then filtered through celite. The filtrate was evcaporated to ca. 2 ml, treated with water (5 ml) and then dropwise with 0.5N aqueous NaOH to pH 7.5. The solution was washed with ether, (10 ml) evaporated under reduced pressure to ca. 2 ml, and the remaining aqueous solution was freeze dried to provide sodium 6-R-6-(α-hydroxybenzyl)-9-0-methyl clavulanate as a pale yellow solid (81 mg): $\nu_{max.}$ (KBr) 1782, 1695 and 1616 $cm^{-1}$; δ ($D_2O$: HOD ≡ 4.60δ) 3.19 (3H, s), 3.66 (1H, d, J 6.5 Hz), 3.94 (2H, d, J 7.5 Hz), 4.7 - 5.0 (2H, m), 5.13 (1H, d, J 6.5 Hz), 5.70 (1H, s), 7.36 (5H, s).

Example 4

Sodium 6-S-6-(α-hydroxybenzyl)-9-O-methyl clavulanate

Methoxymethyl 6-S-6-(α-hydroxybenzyl)-9-O-methyl clavulanate (85 mg) was dissolved in distilled tetrahydrofuran (5 ml) and water (30 ml) was added to the solution. The resulting solution was stirred vigorously and treated dropwise with 0.2N aqueous NaOH, maintaining the pH at 8.5 - 9.5. When the hydrolysis was almost complete (shown by silica t.l.c., eluted with ethyl acetate/hexane 1:1) the pH of the solution was allowed to fall to 7.5. The solution was washed with ether (50 ml) and evaporated under reduced pressure to ca. 2 ml. This remaining aqueous solution was freeze dried to provide sodium 6-S-6-(α-hydroxybenzyl)-9-O-methyl clavulanate as a yellow solid (65 mg): $\nu_{max.}$ (KBr) 1780, 1694 and 1618 $cm^{-1}$: δ ($D_2O$: HOD ≡ 4.60δ) 3.13 (3H, s), 3.7 - 4.0 (2H, m), 4.02 (1H, dd, J 10 and 3 Hz), 4.6 - 4.9 (3H, m), 5.33 (1H, d, J 3 Hz), 7.2 - 7.4 (5H, m).

Example 5



Mixture of benzyl 6-E-benzylidene-9-O-methylclavulanate and benzyl 6-Z-benzylidene-9-O-methylclavulanate

A solution of benzyl 6-R-6-(α-hydroxybenzyl)-9-O-methylclavulanate (100 mg) in dichloromethane (3 ml) was stirred and treated with pyridine (0.4 ml) and then with methanesulphonyl chloride (0.15 ml). After overnight stirring, the solution was washed with 0.1N hydrochloric acid (3 ml), 1N aqueous sodium bicarbonate solution (3 ml) and water (3 ml), dried over $MgSO_4$ and evaporated under reduced pressure. The residue was chromatographed on silica, eluting with ethyl acetate/hexane 1:3, to provide a mixture of benzyl 6-E-benzylidene-9-O-methylclavulanate and benzyl 6-Z-benzylidene-9-O-methylclavulanate as a colourless gum: δ ($CDCl_3$) 3.25 (3H, s), 4.02 (2H, d, J 7.5 Hz), 4.85 (1H, broad t, J 7.5 Hz), 5.18 (1H, broad s), 5.24 (2H, s), 6.07 and 6.33 (1H, 2s of approx. equal intensity), 6.85 and 7.17 (1H, 2s of approximately equal intensity), 7.3 - 8.1 (10H, m).

Example 6



Mixture of methoxymethyl 6-E-benzylidene-9-O-methylclavulanate and methoxymethyl 6-Z-benzylidene-9-O-methylclavulanate

A solution of methoxymethyl 6-R-6-(α-hydroxybenzyl)-9-O-methylclavulanate (200 mg) in dichloromethane (8 ml) was ice-cooled and treated with pyridine (.89 ml) and methane sulphonyl chloride (.31 ml). The solution was stirred overnight at room temperature, washed with 0.1N hydrochloric acid (10 ml), dried over $MgSO_4$ and evaporated under reduced pressure. The residue was chromatographed on silica, eluting with ethyl acetate/hexane 1:3, to provide a mixture of methoxymethyl 6-E-benzylidene-9-O-methylclavulanate and methoxymethyl 6-Z-benzylidene-9-O-methylclavulanate as a colourless gum (105 mg): $\nu_{max.}$ ($CHCl_3$) 1780, 1760(s) and 1680 $cm^{-1}$; δ ($CDCl_3$) 3.23 (3H, s), 3.45 (3H, s), 4.00 (2H, d, J 7 Hz), 4.83 and 4.86 (1H, 2t of approximately equal intensity, J 7 Hz), 5.10 (1H, s), 5.29 (2H, ABq), 6.01 and 6.28 (1H, 2s of approximately equal intensity), 6.78 and 7.10 (1H, 2s of approximately equal intensity), 7.3 - 8.0 (5H, m).

Example 7

Mixture of sodium 6-E-benzylidene-9-O-methylclavulanate and sodium 6-Z-benzylidene-9-O-methylclavulanate

A mixture of methoxymethyl 6-E-benzylidene-9-O-methylclavulanate and methoxymethyl 6-Z-benzylidene-9-O-methylclavulanate (100 mg) was dissolved in tetrahydrofuran (5 ml). Water (40 ml) was added and the mixture was stirred vigorously while 0.2N aqueous sodium hydroxide was added dropwise, maintaining the pH at 9 - 9.5. When the ester hydrolysis was almost complete (shown by silica t.l.c., eluting with ethyl acetate/hexane 1:2), the pH of the solution was allowed to fall to 7.5. The solution was washed with ether (100 ml) and evaporated under reduced pressure to ca. 5 ml. This residual aqueous solution was freeze dried to provide a mixture of sodium 6-E-benzylidene-9-O-methylclavulanate and sodium 6-Z-benzylidene-9-O-methylclavulanate as a pale yellow solid (70 mg): $\nu_{max.}$ (KBr) 1770, 1676 and 1618 $cm^{-1}$ ; $\delta$ ($D_2O$: HOD$\equiv$ 4.60 ) 3.05 and 3.12 (3H, 2s), 3.7 - 4.1 (2H, m), 4.88 (1H, broad s), 5.98 and 6.23 (1H, 2s of approx. equal intensity), 6.70 and 6.88 (1H, 2s of approx. equal intensity), 7.0 - 7.8 (5H, m).

Example 8

Methoxymethyl 6-R-6-(α-hydroxy-2-furylmethyl)-9-0-methylclavulanate

A mixture of tetrahydrofuran (200 ml, distilled from sodium hydride) and hexamethylphosphoramide (20 ml, dried over molecular sieve) was stirred at -70°C under nitrogen and treated with a solution of lithium hexamethyldisilazide in tetrahydrofuran (15.6 ml of a 1M solution). A solution of methoxymethyl 9-O-methylclavulanate (2g) in distilled tetrahydrofuran (10 ml) was added dropwise over 2 minutes, and after a further 15 minutes the mixture was treated dropwise over 2 minutes with a solution of furan-2-carboxaldehyde (.703 ml) in distilled tetrahydrofuran (5 ml). The mixture was stirred at -70°C for a further 40 minutes, treated with a mixture of acetic acid (2.5 ml) and water (10 ml) and warmed to room temperature. Ethyl acetate (200 ml) and saturated brine (200 ml) were added, the mixture was shaken and the layers separated. The organic layer was washed with water (2 x 100 ml), dried over $MgSO_4$ and evaporated under reduced pressure. The residue was chromatographed on silica, eluting with ethyl acetate/hexane 1:2. Appropriate fractions were combined and evaporated to provide methoxymethyl 6-R-6-(α-hydroxy-2-furylmethyl)-9-0-methylclavulanate as a gum (0.27g); $\nu_{max}(CHCl_3)$ 3580, 3370, 1795, 1750 and 1690 $cm^{-1}$; δ($CDCl_3$) 3.26 (3H, s), 3.4-3.8 (5H, m including 3H, s, at 3.43δ), 4.03 (2H, d, J7.5Hz), 4.87 (1H, t, J7.5Hz), 5.1-5.4 (4H, m), 5.89 (1H, s), 6.3-6.5(2H, m), 7.3-7.5 (1H, m).

Example 7

Mixture of sodium 6-E-benzylidene-9-O-methylclavulanate and sodium 6-Z-benzylidene-9-O-methylclavulanate

A mixture of methoxymethyl 6-E-benzylidene-9-O-methylclavulanate and methoxymethyl 6-Z-benzylidene-9-O-methylclavulanate (100 mg) was dissolved in tetrahydrofuran (5 ml). Water (40 ml) was added and the mixture was stirred vigorously while 0.2N aqueous sodium hydroxide was added dropwise, maintaining the pH at 9 - 9.5. When the ester hydrolysis was almost complete (shown by silica t.l.c., eluting with ethyl acetate/hexane 1:2), the pH of the solution was allowed to fall to 7.5. The solution was washed with ether (100 ml) and evaporated under reduced pressure to ca. 5 ml. This residual aqueous solution was freeze dried to provide a mixture of sodium 6-E-benzylidene-9-O-methylclavulanate and sodium 6-Z-benzylidene-9-O-methylclavulanate as a pale yellow solid (70 mg): $\nu_{max.}$ (KBr) 1770, 1676 and 1618 $cm^{-1}$; $\delta$ ($D_2O$: HOD ≡ 4.60) 3.05 and 3.12 (3H, 2s), 3.7 - 4.1 (2H, m), 4.88 (1H, broad s), 5.98 and 6.23 (1H, 2s of approx. equal intensity), 6.70 and 6.88 (1H, 2s of approx. equal intensity), 7.0 - 7.8 (5H, m).

Example 8

Methoxymethyl 6-R-6-(α-hydroxy-2-furylmethyl)-9-0-methylclavulanate

A mixture of tetrahydrofuran (200 ml, distilled from sodium hydride) and hexamethylphosphoramide (20 ml, dried over molecular sieve) was stirred at -70°C under nitrogen and treated with a solution of lithium hexamethyldisilazide in tetrahydrofuran (15.6 ml of a 1M solution). A solution of methoxymethyl 9-O-methylclavulanate (2g) in distilled tetrahydrofuran (10 ml) was added dropwise over 2 minutes, and after a further 15 minutes the mixture was treated dropwise over 2 minutes with a solution of furan-2-carboxaldehyde (.703 ml) in distilled tetrahydrofuran (5 ml). The mixture was stirred at -70°C for a further 40 minutes, treated with a mixture of acetic acid (2.5 ml) and water (10 ml) and warmed to room temperature. Ethyl acetate (200 ml) and saturated brine (200 ml) were added, the mixture was shaken and the layers separated. The organic layer was washed with water (2 x 100 ml), dried over $MgSO_4$ and evaporated under reduced pressure. The residue was chromatographed on silica, eluting with ethyl acetate/hexane 1:2. Appropriate fractions were combined and evaporated to provide methoxymethyl 6-R-6-(α-hydroxy-2-furylmethyl)-9-0-methylclavulanate as a gum (0.27g); $\nu_{max}$ ($CHCl_3$) 3580, 3370, 1795, 1750 and 1690 $cm^{-1}$; δ($CDCl_3$) 3.26 (3H, s), 3.4-3.8 (5H, m including 3H, s, at 3.43δ), 4.03 (2H, d, *J*7.5Hz), 4.87 (1H, t, *J*7.5Hz), 5.1-5.4 (4H, m), 5.89 (1H, s), 6.3-6.5(2H, m), 7.3-7.5 (1H, m).

Example 9

Methoxymethyl 6-(Z-2-furylmethylene)-9-O-methylclavulanate and methoxymethyl 6-(E-2-furylmethylene)-9-O-methylclavulanate

A solution of methoxymethyl 6-R-6-(α-hydroxy-2-furylmethyl)-9-O-methylclavulanate (0.27g) in dichloromethane (10 ml) was ice cooled and treated with pyridine (0.8 ml) and methanesulphonyl chloride (0.23 ml). The ice bath was removed and the solution stirred for 5 hours, washed with 0.5N hydrochloric acid, dried over $MgSO_4$ and evaporated under reduced pressure. The residue was chromatographed on silica, eluting with ethyl acetate/hexane 1:3 then 1:2. Appropriate fractions were combined and evaporated: thus methoxymethyl 6-(Z-2-furylmethylene)-9-O-methylclavulanate and methoxymethyl 6-(E-2-furylmethylene)-9-O-methylclavulanate were separated and obtained as gums.

Less polar geometric isomer (50 mg); $\nu_{max}$ ($CHCl_3$) 1775, 1760, 1690 and 1670 $cm^{-1}$; $\lambda_{max}$ (EtOH) 329 nm (ε 17780); δ($CDCl_3$) 3.26 (3H, s), 3.47 (3H, s), 4.03 (2H, d, J7.5Hz), 4.88 (1H, broad t, J7.5H), 5.11 (1H, broad s), 5.33 (2H, ABq), 7.45 (1H, d, J4Hz), 7.57 (1H, d, J2Hz).

More polar geometric isomer (55 mg): $\nu_{max}$ ($CHCl_3$) 1775, 1760(s) and 1670 $cm^{-1}$; $\lambda_{max}$ (EtOH) 323 nm (ε 22110); δ($CDCl_3$) 3.23 (3H, s), 3.48 (3H, s), 4.03 (2H, d, J7.5Hz), 4.88 (1H, broad t, J7.5Hz), 5.11 (1H, broad s), 5.33 (2H, ABq), 6.36 (1H, s), 6.56 (1H, dd, J3.5 and 2 Hz), 6.81 (1H, d, J3.5 Hz), 6.91 (1H, s), 7.65 (1H, d, J2Hz).

Example 10

Sodium 6-(2-furylmethylene)-9-0-methylclavulanate by hydrolysis of less polar geometric isomer of methoxymethyl 6-(2-furylmethylene)-9-0-methylclavulanate

Methoxymethyl 6-(2-furylmethylene)-9-O-methylclavulanate (less polar geometric isomer, 70 mg) was dissolved in tetrahydrofuran (6 ml), and water (40 ml) added. The resulting solution was stirred vigorously and the pH maintained at 8.5-9.5 by dropwise addition of 0.2N sodium hydroxide solution. When ester hydrolysis was almost complete (shown by tlc, eluted with ethyl acetate/hexane 1:1) the solution was allowed to reach pH 7.5, washed with ethyl acetate (50 ml) and evaporated under reduced pressure to ca 5 ml. This remaining aqueous solution was freeze dried to provide sodium 6-(2-furylmethylene)-9-O-methylclavulanate as a solid: $\nu_{max}$ (KBr) 1766, 1692, 1668, and 1616 $cm^{-1}$; $\delta(D_2O:HOD \equiv 4.80)$, 3.27 (3H, s), 4.0-4.2 (2H, m), 4.87 (1H, t, J7.5Hz), 4.98 (1H, s), 6.11 (1H, s), 6.6-6.7 (1H, m), 6.90 (1H, s), 7.15 (1H, d, J3.5Hz), 7.72 (1H, s); $\lambda_{max}$ ($H_2O$) 331.5 ($\varepsilon$ 16600).

Example 11

Sodium 6-(2-furylmethylene)-9-0-methylclavulanate by hydrolysis of more polar geometric isomer of methoxymethyl 6-(2-furylmethylene)-9-0-methylclavulanate

Methoxymethyl 6-(2-furylmethylene)-9-O-methylclavulanate (more polar geometric isomer, 85 mg) was dissolved in tetrahydrofuran (6 ml), and water (40 ml) wa: added. The resulting solution was stirred vigorously and the pH maintained at 8.5-9.5 by addition of 0.2N sodium hydroxide solution. When ester hydrolysis was almost complete (shown by tlc, eluting with ethyl acetate/hexane 1:1), the solution was allowed to reach pH 7.5, washed wi ethyl acetate (40 ml) and evaporated under reduced pressu

to *ca* 5 ml. This aqueous solution was freeze dried to provide sodium 6-(2-furylmethylene)-9-*O*-methylclavulanate as a solid (50 mg): $\nu_{max}$ (KBr) 1772, 1690(s), 1670 and 1616 $cm^{-1}$; $\delta$($D_2O$: HOD≡4.80) 3.22 (3H, s), 4.03 (2H, d, *J* 7.5 Hz), 4.86 (1H, t, *J*7.5 Hz), 4.98 (1H, s), 6.39 (1H, s), 6.6-6.7 (1H, m), 6.99 (1H, d, *J*3.5Hz), 7.07 (1H, s), 7.78 (1H, s); $\lambda_{max}$ ($H_2O$) 328.5 nm ($\varepsilon$ 20470).

Claims

1. A compound of the general formula I:

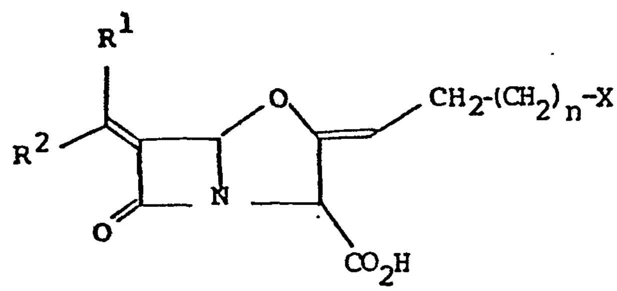

(I)

or a pharmaceutically acceptable salt or *in-vivo* hydrolysable ester thereof

in which

each of $R^1$ and $R^2$, which may be the same or different, denotes a hydrogen atom, an aryl group, or a heterocyclyl group;

X denotes a hydrogen atom, or a hydroxy, substituted hydroxy, mercapto, substituted mercapto, azido, cyano, halo, nitro, isothiocyanato, amino, substituted amino, aryl, or heterocyclyl group, or the residue of a carbon nucleophile; and

*n* denotes 0, 1 or 2.

2. A compound of the general formula IA

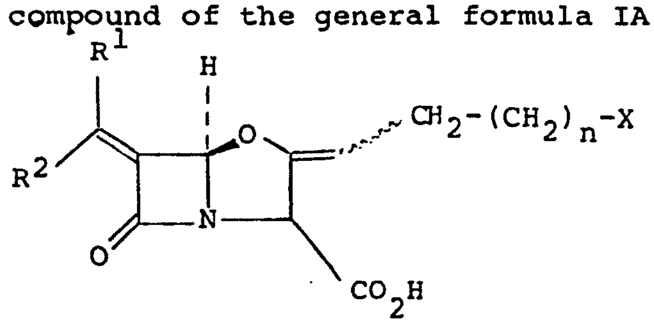

IA

or a pharmaceutically acceptable salt or <u>in-vivo</u> hydrolysable ester thereof

in which $R^1$, $R^2$, X and n are defined as in claim 1.

3. A compound as claimed in claim 1 or claim 2, wherein $R^1$ denotes an aryl group or a heterocyclyl group and $R^2$ denotes a hydrogen atom.

4. A compound as claimed in any one of claims 1 to 3, wherein n denotes 0.

5. A compound selected from:

6-E-benzylidene-9-O-methylclavulanate,

6-Z-benzylidene-9-O-methylclavulanate,

6-E-2'-furylmethylene-9-O-methylclavulanate,

and

6-Z-2'-furylmethylene-9-O-methylclavulanate,

or a pharmaceutically acceptable salt or in-vivo hydrolysable ester thereof.

6. A process for the preparation of a compound of the general formula I as defined in claim 1, or a pharmaceutically acceptable salt or <u>in-vivo</u> hydrolysable ester thereof, which comprises eliminating the elements of a compound of the general formula II:

H-Y II

from a compound of the general formula III:

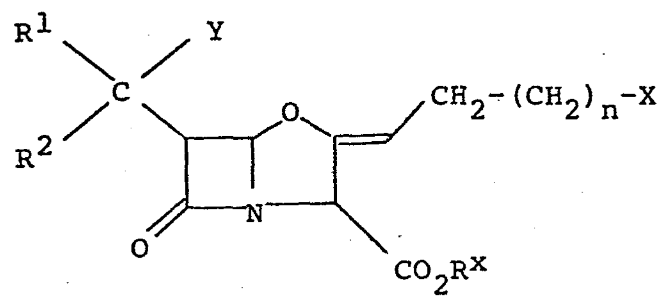

III

in which formulae

$R^1$, $R^2$, X and n are defined as in claim 1,

Y denotes a hydroxy group or a leaving group, and

$R^x$ denotes hydrogen or a carboxyl-blocking group,

any reactive groups in X optionally being protected,

and thereafter if necessary or desired:

(i) removing any protecting group on the group X; and/or

(ii) converting one group X into another group X; and/or

(iii) removing any carboxyl-blocking group $R^x$; and/or

(iv) converting the product into the free acid or into a pharmaceutically acceptable salt or <u>in-vivo</u> hydrolysable ester.

7. A compound of the general formula III:

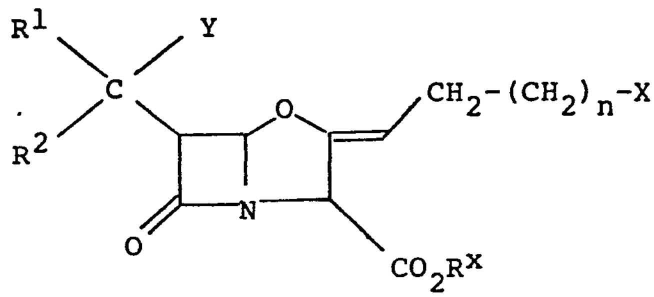

III

in which

$R^1$, $R^2$, X and n are defined as in claim 1, and

Y and $R^x$ are defined as in claim 6.

8. A pharmaceutical composition comprising a compound as claimed in any one of claims 1 to 5 and a pharmaceutically acceptable carrier.

9. A pharmaceutical composition as claimed in claim 8, which additionally comprises a penicillin, cephalosporin or other β-lactam antibiotic.

10. A compound as claimed in any one of claims 1 to 5 for use as a therapeutically active substance.

11. A compound as claimed in any one of claims 1 to 5 for use in the treatment of a bacterial infection.

12. A compound as claimed in any one of claims 1 to 5 for use in the treatment of a bacterial infection in admixture or conjunction with a penicillin, cephalosporin or other β-lactam antibiotic.

European Patent Office

# EUROPEAN SEARCH REPORT

0167050

Application number

EP 85 10 7476

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 041 768 (BEECHAM) * Claims * | 1,6-12 | C 07 D 498/04<br>A 61 K 31/42 //<br>(C 07 D 498/04<br>C 07 D 263:00<br>C 07 D 205:00 ) |
| A | US-A-4 076 826 (B.A. CHRISTENSEN) * Claims * | 7 | |
| A | US-A-4 078 068 (B.G. CHRISTENSEN) * Claims * | 7 | |

TECHNICAL FIELDS SEARCHED (Int. Cl.4)

C 07 D 498/00
C 07 D 499/00
A 61 K 31/00

The present search report has been drawn up for all claims

| Place of search | Date of completion of the search | Examiner |
|---|---|---|
| THE HAGUE | 09-09-1985 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document